# EUROPEAN PATENT APPLICATION

(11) **EP 1 785 166 A1**
(43) Date of publication of application: **16.05.2007**
(21) Application number: 06124114.7
(22) Date of filing: 15.11.2006
(51) Int. Cl.: A62B 23/06, A61M 15/08

(54) **Nasal cavity smog filtration device**

(30) Priority: 15.11.2005 IT RM20050142 U
(71) Applicant: Narciso, Paolo, 00194 Roma (IT)
(72) Inventor: Narciso, Paolo, 00194 Roma (IT)
(74) Representative: Jorio, Paolo

(57) **Abstract**

A nasal cavity filtration device (1) having a hollow, substantially cylindrical supporting structure (2) which is housed inside and adheres to a nasal cavity of a user; one or more transverse filters (3) made of filtering material and closing the supporting structure (2); and a liner (4) located on an inner surface (2a) of the supporting structure (2) to collect harmful particles in the air breathed in.

## Description

The present invention relates to a nasal cavity smog filtration device.

More specifically, the smog filtration device according to the present invention is disposable, and provides for protecting the respiratory tracts mainly against atmospheric pollution.

As is known, concern has frequently been expressed regarding the harmful effect of atmospheric pollution, the major health threats of which include concentrations of sulphur dioxide and fine dust in industry and transport, and pollution by carbon monoxide, ozone, and benzene.

Scientific research leaves no doubt as to the close relationship between the concentration of these pollutants in the air and the occurrence of respiratory disorders.

To combat or even only partly solve the problem, the market currently offers practically no products that are fast, easy, and comfortable to use.

It is an object of the present invention to provide a device designed to prevent inhalation of harmful particles in the air.

According to the present invention, there is provided a nasal cavity filtration device, as claimed in the accompanying Claims.

A non-limiting embodiment of the invention will be described by way of example with reference to the accompanying drawings, in which:
Figure 1 shows a view in perspective of the device according to the present invention;
Figure 2 shows a section of the Figure 1 device.

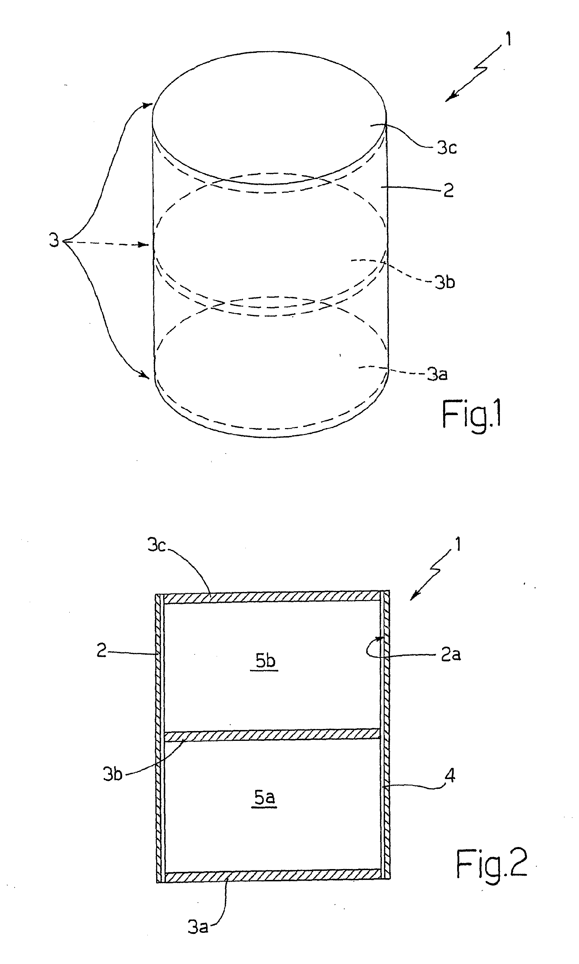

Number 1 in Figures 1 and 2 indicates as a whole the device according to the present invention.

Device 1 comprises a cylindrical supporting structure 2 shaped to fit inside the nasal cavity of a user. Supporting structure 2 is made of soft material, such as latex, covered with an outer material, such as paper, rubber-coated paper, silk, cotton, sponge, gauze silk, or hypoallergenic plastic materials, and has a soft consistency to adhere perfectly to the nasal cavity to ensure perfect sealing and firm seating unaffected by inspiration and expiration.

The supporting structure is also designed to adapt to the nasal pyramid, with no functional impairment of the sebaceous glands or respiratory, olfactory, and phonatory functions.

Device 1 comprises three transverse filters 3 made of cellulose, and which act as a sieve. More specifically, filters 3 may differ from one another, depending on filtration requirements.

Device 1 comprises a liner 4 on the inner surface 2a of supporting structure 2. Liner 4 is electrostatically charged and made of one or more excipients from the group comprising: thymol, essential oils of various woods, essential oil of nutmeg, white Vaseline, lanoline, hydrogenated fatty acid triglycerides, glycerin, polyethylene glycols, and starch; and of one or more active principles from the group comprising: camphor, essential oil of turpentine, menthol, essential oil of eucalyptus, kaolin, and bentonite.

The first filter 3a operates in direct contact with the air to perform a first mechanical filtration. The air issuing from filter 3a is subjected to the action of liner 4, which catches the particles not filtered by filter 3a. The air then flows through the second filter 3b, in which it undergoes a second filtration, and is then subjected again to the action of liner 4. And finally, the air undergoes a third and last filtration through the third filter 3c, which removes further pollutants before the air enters the human body. In other words, two cylindrical, electrostatic filtration chambers 5a and 5b are formed inside supporting structure 2, and are defined laterally by liner 4, and axially by filters 3a and 3b and filters 3b and 3c respectively.

As will be clear from the above description, the device according to the present invention is extremely straightforward and reliable, by combining the sieve filtration of filters 3 with the electrostatic attraction of liner 4.

Obviously, the materials and embodiments may differ from those described above, provided they result in the combined filtration action of the present invention.

## Claims

1. A nasal cavity filtration device (1), **characterized by** comprising a hollow, substantially cylindrical supporting structure (2) which is housed inside and adheres to a nasal cavity of a user; at least one transverse filter (3) made of filtering material and closing said supporting structure (2); and a liner (4) located on an inner surface (2a) of said supporting structure (2) to collect harmful particles in the air breathed in.

2. A filtration device as claimed in Claim 1, **characterized in that** said liner is electrostatically charged.

3. A filtration device as claimed in Claim 1 or 2, **characterized in that** said liner (4) comprises one or more excipients from the group comprising: thymol, essential oils of various woods, essential oil of nutmeg, white Vaseline, lanoline, hydrogenated fatty acid triglycerides, glycerin, polyethylene glycols, and starch; and one or more active principles from the group comprising: camphor, essential oil of turpentine, menthol, essential oil of eucalyptus, kaolin, and bentonite.

4. A filtration device as claimed in one of the foregoing Claims, **characterized in that** said supporting structure (2) is made of latex covered with an outer material from the group comprising paper, rubber-coated paper, silk, cotton, sponge, gauze silk, and hypoallergenic plastic materials.

5. A filtration device as claimed in one of the foregoing Claims, **characterized by** comprising three filters (3), two of which (3a, 3c) are located at the ends of said supporting structure (2), and one of which (3b) is located in the middle of said supporting structure (2), so as to define two filtration chambers (5a, 5b).
